(19) **Europäisches Patentamt European Patent Office Office européen des brevets**

(11) **EP 1 956 031 B1**

(12) **EUROPEAN PATENT SPECIFICATION**

(45) Date of publication and mention of the grant of the patent:
**15.09.2010 Bulletin 2010/37**

(21) Application number: **06817994.4**

(22) Date of filing: **20.11.2006**

(51) Int Cl.:
***C07K 14/575*** (2006.01)

(86) International application number:
**PCT/CU2006/000013**

(87) International publication number:
**WO 2007/059714 (31.05.2007 Gazette 2007/22)**

(54) **NEUROPEPTIDES FOR THE CULTURE OF AQUATIC ORGANISMS**

NEUROPEPTIDE FÜR DIE KULTUR VON WASSERORGANISMEN

NEUROPEPTIDES POUR LA CULTURE D'ORGANISMES AQUATIQUES

(84) Designated Contracting States:
**AT BE BG CH CY CZ DE DK EE ES FI FR GB GR HU IE IS IT LI LT LU LV MC NL PL PT RO SE SI SK TR**

(30) Priority: **22.11.2005 CU 2312005**

(43) Date of publication of application:
**13.08.2008 Bulletin 2008/33**

(73) Proprietor: **CENTRO DE INGENIERIA GENETICA Y BIOTECNOLOGIA (CIGB) Ciudad de la Habana 10600 (CU)**

(72) Inventors:
• **LUGO GONZÁLEZ, Juana, María**
**La Habana 33 600 (CU)**
• **ESTRADA GARCÍA, Mario, Pablo**
**Ciudad De La Habana Playa 10600 (CU)**
• **RODRÍGUEZ MALLON, Alina**
**Ciudad De La Habana 11600 (CU)**
• **CARPIO GONZÁLEZ, Yamila**
**Ciudad De La Habana 10400 (CU)**
• **MORALES ROJAS, Antonio**
**Ciudad De La Habana 10600 (CU)**

• **RODRIGO GONZÁLEZ DE SOSA, Osmany**
**Ciudad De La Habana 17100 (CU)**
• **MORALES FERNÁNDEZ, Reynold**
**Ciudad De La Habana 11600 (CU)**
• **HERRERA MIYARES, Fidel, Francisco**
**Ciudad De La Habana 11600 (CU)**

(74) Representative: **Hatzmann, Martin Vereenigde Johan de Wittlaan 7 2517 JR Den Haag (NL)**

(56) References cited:
**EP-A1- 1 477 181      WO-A-94/26897**

• **MATSUDA K ET AL: "Anorexigenic action of pituitary adenylate cyclase-activating polypeptide (PACAP) in the goldfish: Feeding-induced changes in the expression of mRNAs for PACAP and its receptors in the brain, and locomotor response to central injection" NEUROSCIENCE LETTERS, LIMERICK, IE, vol. 386, no. 1, 23 September 2005 (2005-09-23), pages 9-13, XP004986031 ISSN: 0304-3940**

SEQUENCE LISTING

<110> Center the Genetic Engineering and Biotechnology

<120> NEUROPEPTIDES FOR THE AQUATIC ORGANISMS CULTURE

<130> P84843EP00

<150> PCT/CU2006/000013
<151> 2006-11-20

<150> CU 2005-023120
<151> 2005-11-22

<160> 14

<170> PatentIn ver. 2.1

<210> 1
<211> 29
<212> DNA
<213> Artificial sequence

<220>
<223> Description of the Artificial sequence: primer

<220>
<221> 5'UTR

<222> ()..)

<223> Primer to amplify the complete nucleotide sequence of the GHRH-PACAP gene.


<220>

<221> 5'UTR

<222> (1)..(29)

<400> 1

gcagccatgg ccaaatctag tagagctac        29


<210> 2

<211> 31

<212> DNA

<213> Artificial sequence

<220>

<223> Description of the Artificial sequence: primer


<220>

<221> 3'UTR

<222> ()..)

<223> Primer to amplify the complete nucleotide sequence of the GHRH-PACAP gene.


<220>

<221> 3'UTR

<222> (1)..(31)

<400> 2

ggaattcctt taatggcttg acttcgtaca t        31


<210> 3

<211> 32

<212> DNA

<213> Artificial sequence


<220>

<223> Description of the Artificial sequence: primer


<220>

<221> 5'UTR

<222> ()..)

<223> Primer to amplify the PACAP gene.


<220>

<221> 5'UTR

<222> (1)..(32)


<220>

<221> 5'UTR

<222> (1)..(32)

<400> 3

catccatatg cactcggacg gcattttcac gg        32


<210> 4

<211> 38

<212> DNA

<213> Artificial sequence

<220>

<223> Description of the Artificial sequence: primer


<220>

<221> 3'UTR
<222> ()..)
<223> Primer to amplify the PACAP gene.
<220>
<221> 3'UTR
<222> (1)..(38)
<400> 4
cgggatcctt atttgtttct aaacctctgt ctgtacct          38

<210> 5
<211> 22
<212> DNA
<213> Artificial sequence
<220>
<223> Description of the Artificial sequence: primer

<220>
<221> 5'UTR
<222> (1)..(22)
<223> Primer to amplify the PACAP gene for its expression in Pichia pastoris.
<400> 5
cactcggacg gcattttcac gg          22

<210> 6
<211> 31
<212> DNA
<213> Artificial sequence
<220>
<223> Description of the Artificial sequence: primer
<220>
<221> 3'UTR
<222> (1)..(31)
<223> Primer to amplify the PACAP gene for its expression in Pichia pastoris.

<400> 6
actagtttat ttgtttctaa acctctgtct g          31

<210> 7
<211> 30
<212> DNA
<213> Artificial sequence
<220>
<223> Description of the Artificial sequence: primer

<220>
<221> 5'UTR
<222> (1)..(30)
<223> Primer to amplify the PACAP gene for its expression in Pichia pastoris.

<400> 7
ccatgggaca ctcggacggc attttcacgg          30

<210> 8
<211> 727
<212> DNA
<213> Clarias gariepinus

<220>

<210> gene
<222> (1)..(727)
<223> Nucleotide sequence of the GHRH-PACAP gene
<400> 8

```
atggccaaat ctagtagagc tactttggct ctgctcatct acgggatctt aatgcgctac 60
acgcccaatg cacacccatc ggaatgggct tccccaatat gaggctagaa aacgacgtgt 120
tcggggacga gggaaactcg ttaagtgagc tgtcctacga gccggacacg atgagcgcgc 180
gcagtgctcc agccctccct gaagacgcat acacactgta ctacccgccc gagagaagag 240
ccgaaacgca tgcagacgga ttgttagata gagccttgag ggacatcctg gttcagttat 300
cagcccgaaa atatctgcat tctctgacgg cagttcgcgt aggtgaggaa gaagaggatg 360
aagaggactc ggagccactg tcgaagcgcc actcggacgg cattttcacg gacagctaca 420
gccgctaccg gaaacaaatg gccgtaaaaa aataccttgc agcagtgctg ggaagaaggt 480
acagacagag gtttagaaac aaaggacgcc gctttgctta tttgtagcgg ataggaagaa 540
aaggaaagaa agaaaaaaac gcgagagaga gagagagaga gagaaataga gcaactgccc 600
tcccttgtgt ccattcaatc atacagtcag aagtctggta tctaacttaa cactgagcag 660
tcagtcggtg gatctcgcct gtgttctttt aaacatgtat tttatgtacg aagtcaagcc 720
attaaag                                                          727
```

<210> 9
<211> 86
<212> PRT
<213> clarias gariepinus

<220>
<221> PROPEP
<222> ()..()
<223> GHRH-PACAP aminoacid sequence
<400> 9

```
Met His Ala Asp Gly Leu Leu Asp Arg Ala Leu Arg Asp Ile Leu Val
 1               5                  10                  15
Gln Leu Ser Ala Arg Lys Tyr Leu His Ser Leu Thr Ala Val Arg Val
                20                  25                  30
Gly Glu Glu Glu Glu Asp Glu Glu Asp Ser Glu Pro Leu Ser Lys Arg
                35                  40                  45
His Ser Asp Gly Ile Phe Thr Asp Ser Tyr Ser Arg Tyr Arg Lys Gln
        50                  55                  60
Met Ala Val Lys Lys Tyr Leu Ala Ala Val Leu Gly Arg Arg Tyr Arg
 65                  70                  75                  80
Gln Arg Phe Arg Asn Lys
                85
```

<210> 10
<211> 114
<212> DNA
<213> Clarias gariepinus

<220>
<221> gene
<222> (1)..(114)
<223> Nucleotide sequence of the PACAP gene
<400> 10

```
cactcggacg gcattttcac ggacagctac agccgctacc ggaaacaaat ggccgtaaaa 60
```

```
aaataccttg cagcagtgct gggaagaagg tacagacaga ggtttagaaa caaa      114
```

<210> 11
<211> 114
<212> DNA
<213> Tilapia nilotica

<220>
<221> gene
<222> (1)..(114)
<223> Nucleotide sequence of the PACAP gene
<400> 11


cactcggacg gcattttcac ggacagctac agccgctacc ggaaacaaat ggcagtaaaa 60
aagtatcttg cagcagtgct gggaagaagg tacagacaga ggtttagaaa caaa       114


<210> 12
<211> 40
<212> PRT
<213> Clarias gariepinus

<220>
<221> PEPTIDE
<222> (1)..(40)
<223> Pichia pastoris recombinant PACAP (aminoacid sequence)
<400> 12


Met Gly His Ser Asp Gly Ile Phe Thr Asp Ser Tyr Ser Arg Tyr Arg
1               5                   10                  15
Lys Gln Met Ala Val Lys Lys Tyr Leu Ala Ala Val Leu Gly Arg Arg
                20                  25                  30
Tyr Arg Gln Arg Phe Arg Asn Lys
                35                  40


<210> 13
<211> 39
<212> PRT
<213> Claria gariepinus

<220>
<221> PEPTIDE
<222> (1)..(39)
<223> E.coli recombinant PACAP (aminoacid sequence)
<400> 13


Met His Ser Asp Gly Ile Phe Thr Asp Ser Tyr Ser Arg Tyr Arg Lys
1               5                   10                  15
Gln Met Ala Val Lys Lys Tyr Leu Ala Ala Val Leu Gly Arg Arg Tyr
                20                  25                  30
Arg Gln Arg Phe Arg Asn Lys
                35


<210> 14
<211> 38
<212> PRT
<213> Clarias gariepinus

<220>
<221> PEPTIDE

**EP 1 956 031 B1**

<222> (1)..(38)
<223> Pichia pastoris recombinant PACAP (aminoacid sequence without modifications)
<400> 14

```
His Ser Asp Gly Ile Phe Thr Asp Ser Tyr Ser Arg Tyr Arg Lys Gln
 1               5                  10                  15
Met Ala Val Lys Lys Tyr Leu Ala Ala Val Leu Gly Arg Arg Tyr Arg
                20                  25                  30
Gln Arg Phe Arg Asn Lys
```

35

1

**Description**

**Technical field**

The present invention is related to the field of the farming biotechnology, specifically with the use of the Pituitary Adenylate Cyclase-Activating Polypeptide in the aquatic organism culture. When the peptide is applied to aquatic organisms by immersion, injection or as a feed additive, an increase in the appetite of these organisms, a greater rate of growth and survival, a superior immune activity and an increase of the prolactin release is obtained.

**Previous art**

The Pituitary Adenylate Cyclase-Activating Polypeptide (PACAP) was isolated for the first time in 1989 from bovine hypothalamus and was demonstrated that its capacity to stimulate the growth hormone secretion is through the adenylate cyclase enzyme activation. (Miyata et al. (1989) Isolation of a novel 38 residue hypothalamic polypeptide which stimulates adenylate cyclase in pituitary cells. Biochem. Biophys. Res. Commun. 164:567-574). PACAP belongs to the peptide family that includes secretin, glucagon and intestinal vasoactive peptide (Arimura and Shioda (1995) Pituitary adenylate cyclase-activating polypeptide (PACAP) and its receptors: Neuroendocrine and endocrine interaction. Front. Neuroendocrinol. 16:53-88). In mammals, the precursors of the PACAP and the Growth Hormone Releasing Hormone (GHRH) are codified by two different genes (Hosoya et al. (1992) Structure of the human pituitary adenylate cyclase-activating polypeptide (PACAP) gen. Biochim. Biophys. Acta. 1129:199-206), just like in the all submammalian species studied to date (birds, reptiles and fish), GHRH and PACAP peptides are codified by a same gene and are contained in a same precursor (Montero et al. (2000) Molecular evolution of the growth hormone-releasing hormone/ pituitary adenylate cyclase-activating polypeptide gene family. Functional implication in the regulation of growth hormone secretion. Journal of Molec. Endocrinol. 25:157-168). The PACAP gene is expressed fundamentally in the central and peripheral nervous system, in the nervous fibers innerving the eyes, in the respiratory tract, the salivary glands, in the gastrointestinal tract, in the reproductive system organs, in the pancreas and in the urinary tract. It is also synthesized in the adrenal glands, in the gonads and in immune cells (Sherwood et al. (2000) The origin and function of the Pituitary Adenylate Cyclase-Activating Polypeptide (PACAP)/Glucagon Superfamily. Endocrine Review 21:619-670). PACAP displays different biological functions, which is consistent with its diverse distribution in different tissue and with its hypophysiotropic, neurotransmitter, neuromodulate and vasoregulate activity. (Chatterjee et al. (1997) Genomic organization of the rat pituitary adenylate cyclase-activating polypeptide receptor gene. Alternative splicing within the 59-untranslated region. J. Biol. Chem. 272:12122-12131).

It is involved in the regulation of the cellular division, differentiation and cell death (Sherwood et al. (2000) The origin and function of the Pituitary Adenylate Cyclase-Activating Polypeptide (PACAP)/Glucagon Superfamily. Endocrine Review 21:619-670).

PACAP stimulates the growth hormone (GH) release. The effect of the peptide on the GH release has been demonstrated *in vitro* in several species of mammals, birds, amphibians (Hu et al. (2000) Characterization and messenger ribonucleic acid distribution of a cloned pituitary adenylate cyclase-activating polypeptide type I receptor in the frog Xenopus laevis brain. Endocrinol. 141:657-665) **and fish** (Anderson L. L. et al. (2004) Growth Hormone Secretion: Molecular and Cellular Mechanisms and In Vivo Approaches. Society for Experim. Biol. and Med. 229:291-302). There are few studies about the effect of PACAP in the GH secretion and release, *in vivo.* To date it is known that this peptide increases *in vivo* the levels of the GH in plasma of rats (Jarr et al. (1992) Contrasting effects of pituitary adenylate cyclase activating polypeptide (PACAP) on in vivo and in vitro prolactin and growth hormone release in male rats. Life Sci. 51: 823-830) **and in bovine plasma** (Radcliff et al. (2001) Pituitary adenylate cyclase-activating polypeptide induces secretion of growth hormone in cattle. Domestic. Animal. Endocrinol. 21:187-196), whereas it does not produce this effect in ewes (Sawangjaroen and Curlewis (1994) Effects of pituitary adenylate cyclaseactivating polypeptide (PACAP) and vasoactive intestinal polypeptide (VIP) on prolactin, luteinizing hormone and growth hormone secretion in the ewe. J. Neuroendocrinol. 6:549-555) **and humans** (Chlodera et al. (1996) Effects of intravenously infused pituitary adenylate cyclase activating polypeptide on adenohypophyseal hormone secretion in normal men. Clin. Neuroendocrinol. 64: 242-246)..

These findings suggest that, in mammals, the peptide effect on the GH secretion varies from species to species (Anderson et al. (2004) Growth Hormone Secretion: Molecular and Cellular Mechanisms and In Vivo Approaches. Society for Experim. Biol. and Med. 229:291-302).

Until now, no studies exist in fish, *in vivo,* that show the PACAP function in the GH regulation. In addition, no antecedents of the use of this peptide in the appetite stimulation in aquatic organisms exist. In crustaceans no evidence exists , so far, of the presence of this peptide and the cascade of signals that regulate the growth in these organisms is not known.

PACAP stimulates in addition the prolactin release by the pituitary cells in mammals (Ortmann et al. (1999) Interactions of ovarian steroids with pituitary adenylate cyclase-activating polypeptide and GnRH in anterior pituitary cells. Eur. J. Endocrinol. 140:207-214**).** It promotes melanotropin α-melanocyte-stimulating hormone (MSH) release by the melanotrophic cells of the pituitary. **(**Vaudry et al. (2000) Pituitary adenylate cyclase-activating polypeptide and its receptors: from structure to functions. Pharmacol. Rev.s 52:269-364**).**

In fish there are no studies that show the activity of this peptide in the prolactin release, *in vivo,* nor are there findings about its effect in the development of the fish color.

In mammals, the function of PACAP on the immune system function is very well characterized and there are several patents that describe its use in humans as an immunological response modulator. Until now there are no antecedents in the literature that explain the function of PACAP on the immune system in aquatic organisms.

The gene coding for PACAP from several vertebrates' species and from a protochordate (tunicate) has been cloned. In fish it has been isolated from some species of salmon and from catfish **(**Sherwood et al. (2000) The Origin and Function of the Pituitary Adenylate Cyclase-Activating olypeptide (PACAP)/Glucagon Superfamily Endocrine Reviews 21(6):619-670**, goldfish (**Leung et al. (1999) Molecular cloning and tissue distribution of in pituitary adenylate cyclase-activating polypeptide (PACAP) the goldfish. Rec. Progr. Mol. Comp. Endocrino/. 338-388**), zebrafish (**Fradinger and Sherwood (2000) Characterization of the gene encoding both growth hormone-releasing hormone (GRF) and pituitary adenylate cyclase-activating polypeptide. Mol. and Cell. Endocrinol. 165:211-219**), trucha (Krueckl and Sherwood. (2001) and trout (**Krueckl and Sherwood. (2001) Developmental expression, alternative splicing and gene copy number for the pituitary adenylate cyclase-activating polypeptide (PACAP) and growth hormone-releasing hormone (GRF) gene in raibow trout Molec. and Cell. Endocrinol. 182:99-108**).** The patent US 5695954 protects the isolation and purification of the genes nucleotide sequences that codes for the fish GHRH-PACAP polypeptide, as well as, vectors and hosts that express these sequences with the objective of being used to increase the growth in fish, via transgenesis, introducing the mentioned genetic constructs in fertilized fish eggs. It also protects a method for detecting transgenic fish that contain these sequences.

In this patent specifically the sequences of the genes are reported that code for the GHRH-PACAP polypeptide of the species *Oncorhynchus Nerka, Clarias macrocephalus* and *Acispenser transmontanus.*

In the present invention, variants of the exact amino acid sequence of PACAP were used with N- terminal modifications, obtained in our laboratory for *Clarias gariepinus* and *Oreochromis niloticus* species. These variants were used as growth stimulators in a non-transgenic manner, by administration by immersion expressed in the supernatant of culture of E. *coli* and *P. pastoris,* without previous purification of them. Unexpectedly, we found that these variants are able, in these conditions, to promote a significant increase of the immunological activity in these organisms and to elevate the prolactin concentration in serum. These properties of the peptide have not been described thus far for aquatic organisms.

Several authors have reported a stimulating effect of growth in fish by administration via immersion of recombinant growth hormone. However, in practice, the direct use of growth hormone is subject to many regulatory requirements, so does the use of transgenic fish that overexpress growth hormone or a growth hormone releasing factor.

The present invention describes a non transgenic methodology to increase growth and improve the immune system of aquatic organisms including invertebrates. Nowadays, aquatic organisms are an important source of proteins, but the capture in its natural form is exploited at the maximimum limit. For this reason, culturing is necessary to increase the production **(**Pullin et al.; Conference Proceeding 7, 432 p. International Center for living Aquatic Resources Management. Manila, Philippines. 1982, ISSN 0115-4389**).**

To increase the efficiency culturing these organisms, by growth stimulation, the increase of the survival and improved quality of the larvae remains a major problem to solve in aquaculture.

**Summary of the Invention**

The present invention solves the above problem by providing variants of Pituitary Adenylate Cyclase-Activating Polypeptide with the amino acids sequences identified as SEQ ID No 12, 13 and 14, which increased the rate of growth of aquatic organisms, including invertebrate organisms, in a short period of time, which is of vital importance for aquaculture. These peptides also increase the survival of fish larvae and crustaceans of commercial interest when they are applied by immersion or as feed additive. They stimulate the immune activity in these organisms and the appetite, the development of the color of the skin and the prolactin release.

In a preferred embodiment of the present invention, the variants of the neuropeptide PACAP are applied to fish or crustaceans by periodic injections with intervals of 3 days in a concentration of 0.1 μg/g of animal weight, by immersion baths with intervals of 1 to 4 days in fresh water or sea water with a peptide concentration between 100 to 200 μg/per liter of water and as feed additive in a concentration of 5mg/Kg of formulated feed. In all cases a significant increase of growth and a superior immune activity is obtained.

The use of the variants of the neuropeptide PACAP offers advantages because of its small size of approximately 5 KDa, which is better absorbed through the skin and mucous of these organisms when it is applied by immersion, which is a method of administration that present cost and manipulation advantages for the aquaculture and with a low index of contamination. In addition, its signals transduction mechanism begins with the activation of an enzyme (adenyl cyclase) and not through the activation of a hormone, and its growth hormone releasing activity is weak in mammals, including humans, Therefore, its use provides better public perception and less regulatory requirements.

Other advantages of the invention are its capacity to stimulate the innate and adaptive immune activity in fish and to increase the resistance to infections by pathogenic agents.

In another embodiment of the present invention, the variants of PACAP were provided to aquatic organisms, such as tilapia *Orechromis sp,* catfish *Claria sp,* salmon *Salmon sp.* and shrimps *Penaus sp.*

In another preferred embodiment of the present invention, the variants of the PACAP are provided to fish or crustaceans to prevent or to treat infections by pathogenic agents.

As a result of this work, an embodiment of the present invention describes a composition for the treatment of fish or crustaceans in culture to stimulate their growth and to increase their resistance against diseases, as well as for preventive or therapeutic treatment of an infection by pathogenic agents, all this with the objective to improve the productivity.

**Brief description of the Figures:**

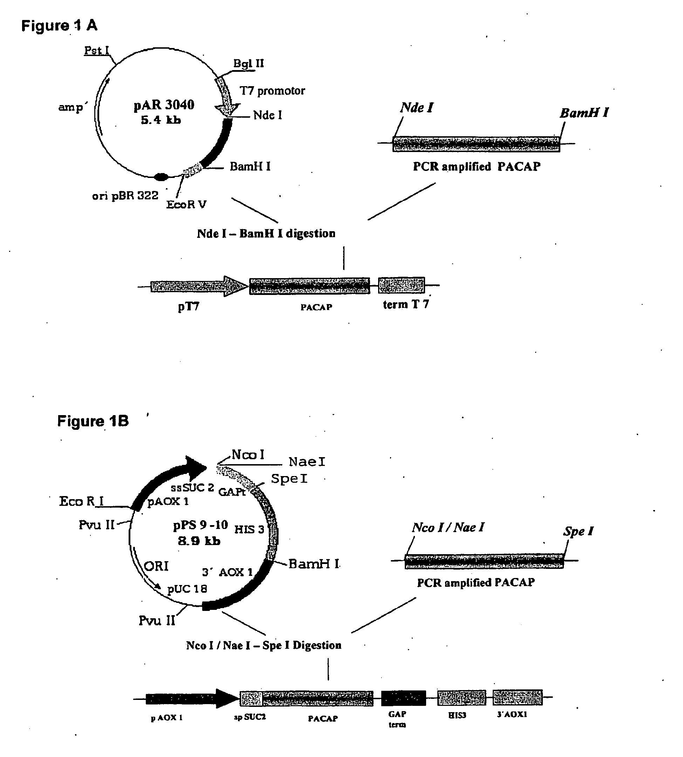

**Figure 1.** Cloning strategy of the peptide of interest in the bacterial expression vectors (Fig. 1A) and in the yeast expression vectors (Fig. 1B).

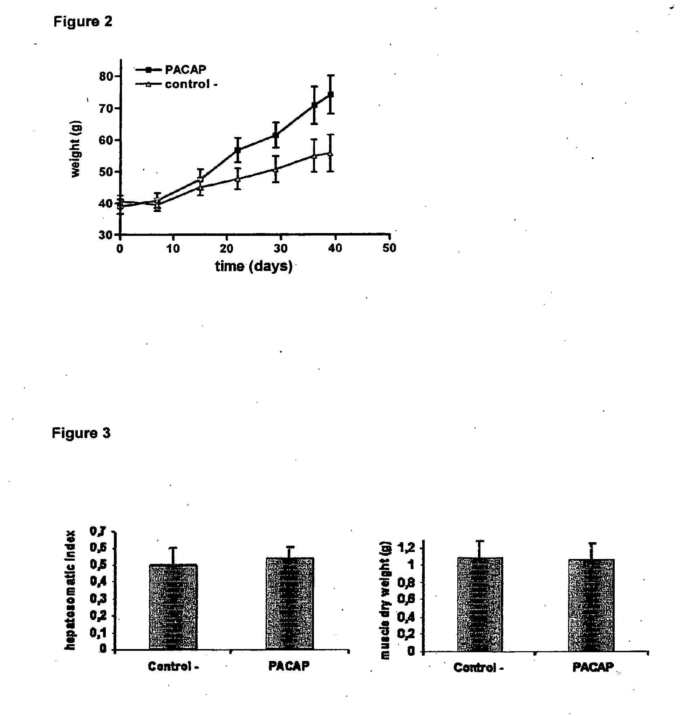

**Figure 2.** Growth stimulation experiment in juvenile *Claria gariepinus* by the intraperitoneal injection of the recombinant PACAP, purified by affinity chromatography, at the dose of 0.1μg/g of animal weight. The graph represents the average body weight of the PACAP treated group compared with the group treated with a placebo during the experiment.

**Figure 3.** Growth stimulation experiment in juvenile *Claria gariepinus* by the intraperitoneal injection of the recombinant PACAP, purified by affinity chromatography, at the dose of 0.1μg/g of animal weight. The graph represents the average of the hepatosomatic index and muscle dry weight of the PACAP treated group compared with the group treated with placebo..

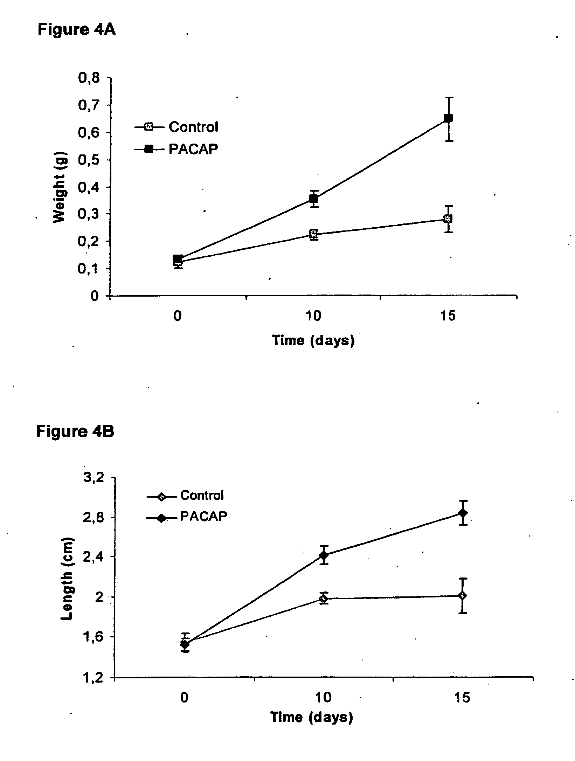

**Figure 4.** Growth stimulation experiment in tilapia larvae by application through immersion in the supernatant of ruptured *E. coli* containing the recombinant PACAP, at the dose of 100 μg /liter of water. Graphic 4A and 4B show the average body weight in grams and length in centimeters of the fish in the group treated with PACAP compared to the group treated with placebo, during the experiment.

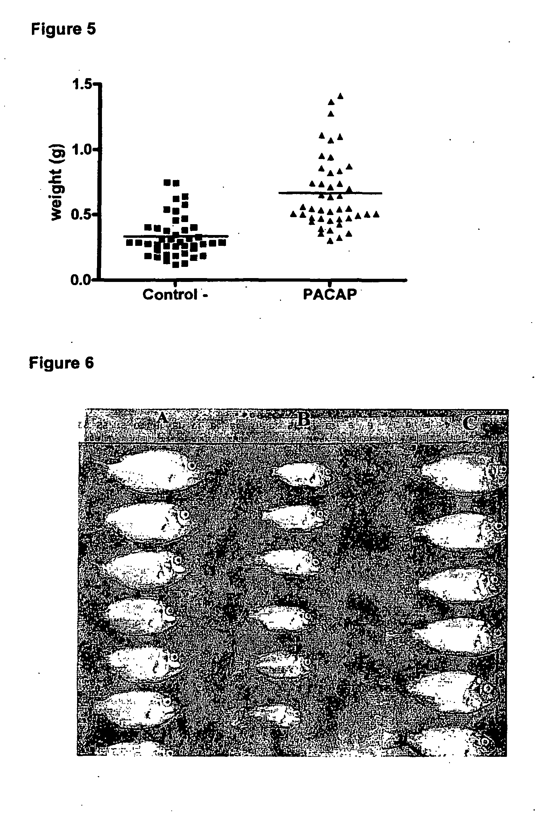

**Figure 5.** Growth stimulation experiment in tilapia larvae by application through immersion in the supernatant of ruptured *E. coli* containing the recombinant PACAP, at the dose of 100 μg /liter of water. The graph represents the weight in grams of fish treated with PACAP and fish treated with placebo at 22 days after the beginning of the treatment.

**Figure 6**. Growth stimulation experiment in tilapia larvae by application through immersion in the supernatant of ruptured *E. coli* containing the recombinant PACAP, at the dose of 100 μg /liter of water. The picture shows the difference in length 30 days after the last immersion bath, of the PACAP treated fish (A and C) compared to the placebo-treated group (B).

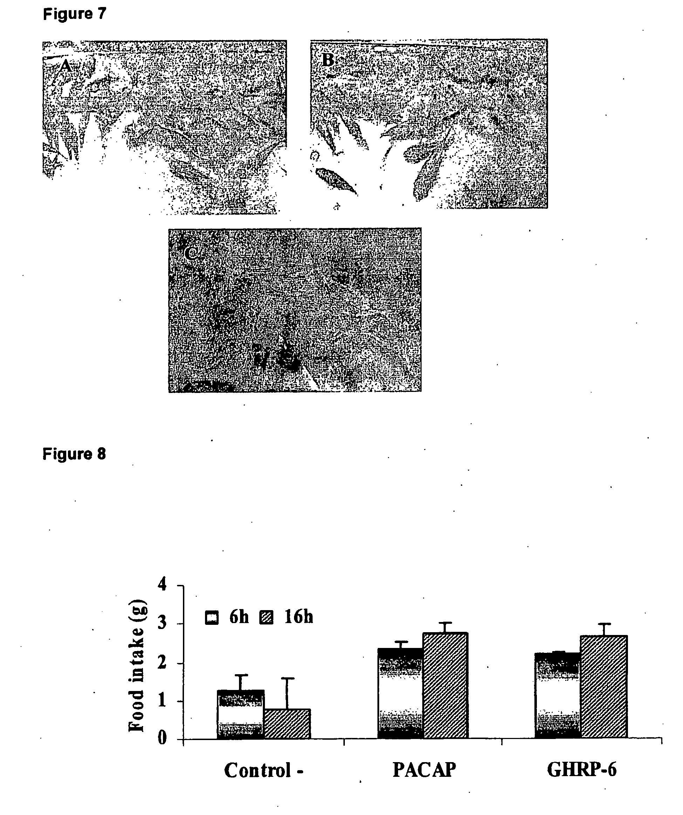

**Figure 7.** Growth stimulation experiment in tilapia larvae by application through immersion in the supernatant of ruptured *E. coli* containing the recombinant PACAP, at the dose of 100 μg /liter of water. The picture shows the earlier development of the fish colors in the PACAP treated fish (A and B) compared to the placebo group (C).

**Figure 8.** Experiment to evaluate the effect on appetite of PACAP recombinant purified by affinity chromatography, in juvenile tilapia species Orechromis niloticus, when applied at doses of 0.5 mg per gram of animal weight. The graph shows the average amount of food ingested by fish in both groups at 6 hours and 22 hours after the beginning of the treatment.

**Detailed description of particular embodiments / Examples**

**Example 1: Construction of vectors for the expression of intracellular PACAP in *E. coli* and for the extracellular form in the supernatant of *P. pastoris* culture.**

The gene coding for PACAP of *Claria gariepinus* was isolated by Polymerase Chain Reaction using the sequence previously obtained by RT-PCR of the gene which encodes the neuropeptide GNRH-PACAP of this species. We used the specific oligonucleotides corresponding to the sequences SEQ ID No 1 and SEQ ID No 2 to obtain the complete sequence of the gene which codes for the polypeptide GHRH-PACAP including the signal sequence, and specific oligonucleotides SEQ ID No 3 and SEQ ID No 4 to amplify the exact gene PACAP with the enzymatic restriction sites required for cloning into an *E. coli* expression vector..

The tilapia PACAP gene was isolated similarly by using the specific oligonucleotides SEQ ID No 3 and SEQ ID No 4. The present invention constitutes the first report of the isolation of this gene in tilapia.

The gene which codes for PACAP was cloned in the *E. coli* expression vector pAR 3040 using the restriction sites NdeI and BamHI (Figure 1A). We selected one of the recombinant clones to transform the *E.coli* BL21D3 strain and to induce the expression of the gene under the regulation of the T7 promoter, using 0.5 mM IPTG as inducer.

The gene expression was carried out to 28°C during 5 hours. The expression of the recombinant PACAP and its integrity was checked by Mass Espectrometry.

For the construction of vectors for expression of PACAP in *P. pastoris,* we used the expression vectors pPS9 and pPS10. We used the specific oligonucleotides SEQ ID No 7 and SEQ ID No 6 for the cloning in pPS9 and the oligonucleotides SEQ ID No 5 and SEQ ID No 6 for the cloning in pPS10. We used the restriction sites NcoI and SpeI for the cloning in pPS7. In this cloning approach, a methionine and a glycine was added at the terminal amino acid of the protein of interest. For the cloning in pPS10 we used the restriction sites NaeI and SpeI,. In this cloning strategy, no amino acids are added to the protein of interest (Figure 1B).

The plasmids were linearized before transforming the MP36 strain of *P. pastoris.* The transformation was carried out by electroporation. The MP36 strain is an auxotrophic mutant his3 which acquired a His$^+$ phenotype after transformation.

The transformed clones were identified by Dot Blot. Using Southern Blot technique, it was determined in which transformants the integration had occurred by the replacement of the gene *AOX*1 of *P. pastoris* by the expression cassette of the recombinant plasmid. This integration event produces a Mut$^s$ (low levels of methanol utilization) and His$^+$ phenotype.

*Pichia pastoris* secretes low levels of proteins by itself and its culture medium does not need proteins as supplements, so you can expect a heterologous protein that is secreted to the extracellular medium, constituting a majority of the total protein in the medium (more than 80%) (Tschopp et al.; Bio/Technology 1987, 5: 1305-1308; Barr et al.; Pharm. Eng. 1992, 12: 48-51). The expression of the PACAP in P. *pastoris* was carried out in bioreactors of 5L with the addition of methanol to the culture.

**Example 2. Growth stimulation experiment in juvenile *Claria gariepinus,* determination of the hepatosomatic index and fish muscle dry weight.**

We used 18 catfish of the *Clarias gariepinus* species, without sex distinction, of approximately the same age and with average body weight of 30 to 40 grams.

Two experimental groups were conformed, each one consisting of nine individuals. The groups were acclimated in separated tanks, with constant water recirculation, at a temperature of 28°C and with a constant photoperiod of 14 hours light and 10 hours of darkness. The animals were fed 2 times per day, with rations equivalent to 5% of the total body weight in each tank. The animals were identified before the experiment. One of the groups was treated with semi-purified PACAP (70% of purity) SEQ ID No. 13, whereas the other group, used as a placebo, was treated with *E.coli* proteins contained in PBS 1X (*E. coli* proteins were obtained by the same purification procedure as the peptide of interest, with equivalent amount of contaminants present in the purified PACAP sample). The PACAP treated fish were intraperitoneally injected with a dosis of 0.1 μg of the peptide per gram of the animal body weight, 2 times per week. The placebo group was injected similarly with a placebo sample. 22 days after the beginning of the experiment, the animals injected with PACAP in the peritoneal cavity, showed a significant increase ($p < 0.05$) of the body weight compared to the animals injected with a placebo (Figure 2).

The hepatosomatic index and the muscle dry weight were determined to demonstrate that the increase in the fish corporal body weight was not due to the increase of the size of the organs, or to the increase of the water content in the muscle.

No significant differences between hepatosomatic index and muscle dry weight values of the experimental groups were observed (Figure 3).

Similar results were obtained when the recombinant PACAP of the sequence SEQ ID No. 12 was used.

**Example 3. Experiment of growth stimulation, of the resistance to pathogenic agents and of the prolactin release, in tilapia larvae by application through immersion baths with the supernatant of ruptured *E. coli* containing the recombinant PACAP.**

We made an experiment to evaluate the function of the *Claria gariepinus* recombinant PACAP present in the supernatant of ruptured *E. coli* in the tilapia larvae growth.

Two experimental groups were formed of 60 larvae in each group, one group was treated with the PACAP with the sequence SEQ ID No. 13 and one placebo group. Larvae from each group were acclimated in separate channels in separated tanks of 80L, at a temperature of 28°C and with a constant photoperiod of 14 hours light and 10 hours of darkness and the animals were fed with an amount of feed according to the formula: Amount of food = # of animals X average weight (g) X 40% /100. The treatments were carried out by immersion in a volume of 2L, three times a week during 20 days, approximately 200 µg of PACAP was used for each immersion bath, for one hour.

Results that were obtained within 10 days after initiation of the experiment, the PACAP-treated group showed an increase in body weight and length which was statistically higher than the placebo group (p <0.01), 15 days after initiation of the experiment, the difference between the treatments was highly significant (p <0.001) (Table 1 and Figure 4A and 4B). For 20 days of immersion baths treatment, the differences between the PACAP-treated group and the placebo group remained highly significant (p <0.001) (Figure 5).

**Table 1. Weight and length of the tilapia larvae at 10 days and 15 days after the beginning of the experiment.**

| | Weight (g) | | Length (cm) | |
|---|---|---|---|---|
| **Treatment** | **10 days** | **15 days** | **10 days** | **15 days** |
| **PACAP** | $0.3536 \pm 0.0879$ | $0.6458 \pm 0.2399$ | $2.41 \pm 0.2726$ | $2.84 \pm 0.3627$ |
| **Placebo** | $0.2221 \pm 0.0565$ | $0.2785 \pm 0.1438$ | $1.98 \pm 0.1687$ | $2.01 \pm 0.5174$ |
| Weight and length are shown as mean $\pm$ S.D | | | | |

It was observed that the effect on growth was maintained over time, because 30 days after the last immersion bath (held 20 days to launch the experiment) differences in body weight and length between the animals of groups test were highly significant (p <0.01) (Figure 6).

In addition, it was observed that the fish treated with peptide developed skin coloration earlier (Figure 7).

In this experiment we also studied the presence of the cutaneous protozoo *Trichodina sp,* 10 animals were selected at random from each experimental group and the invasion intensity by this pathogenic agent was determined. The values of invasion intensity were determined according to the equation:

$$\text{(I: \# total of parasites per fish) } I = \sum N / n\text{-}F_0 \quad y \quad E = n\text{-}F_0 \times 100 / n$$

wherein:

I: (average invasion intensity)
E: (# parasite fish from the total)
$\sum N$: (total of found parasites)
$F_0$: (number of non parasite fish)
n: (number of analyzed fish)

The PACAP treated fish showed an invasion intensity (average of I= 2.20) by the protozoo *Trichodinas sp* significantly inferior (p< 0.01) compared with the group treated with placebo presenting a mean value of I= 5.56.

The fish were treated by immersion bath 45 days of the beginning of the experiment under the same conditions previously described and 24 hours after the treatment blood was extracted. The concentration of prolactin in the serum of each fish (sample of 10 animals per group) was measured by Western blot and ELISA. A polyclonal antibody anti tilapia prolactin was used for these assays. We observed in the fish of the group treated with the peptide statistically higher concentrations of serum prolactin compared with the placebo group p <0.01 (Table 2). These are very attractive results in the commercial aquatic organisms, as it is the case with salmons, which have a life cycle in fresh water and sea water and in which the prolactin performs an important function in the osmoregulation.

**Table 2: Prolactin concentration (ng/mL) in the fish serum at 45 days after the begining of the experiment.**

| Concentración de prolactina | GRUPO TRATADO PACAP | GRUPO PLACEBO |
|---|---|---|
| ng/mL | $36.860 \pm 2.695*$ | $15.745 \pm 1.362$ |
| The concentration is shown as the mean $\pm$ S.D | | |

**Example 4. Experiment to evaluate the effect of recombinant PACAP on the appetite of juvenile tilapias of the *Orechromis niloticus* species.**

Until now, the biological effects of the PACAP in the appetite, in fish, have not been studied. In general, the activity of these peptides on the appetite in the submammalian vertebrate organisms has scarcely been characterized (Jensen, 2001, Regulatory peptides and control of food intake in non-mammalian vertebrates. Comp. Biochem. And Phisiol. Part A 128:471-479).

To analyze the effect of the PACAP in the fish appetite, we used juvenile tilapia of the *Oreochromis niloticus* species, selected at random regardless of sex and approximately the same body weight. Three experimental groups were formed, of 3 individuals each one (3 tilapias per individual). The groups were acclimated in separated tanks with constant water recirculation, at a temperature of 28°C and with a photoperiod of 14 hours of light and 10 hours of darkness.

A group was treated with PACAP (previously obtained from our laboratory, with a purity of 87%) with SEQ ID No. 13, by intraperitoneal injection (0.5 $\mu$g/g of the animals body weight). The second group was treated with the GHRP-6 (Lipotec, S.A, Barcelona, Spain) using the same method of administration at a dose of 0.1 $\mu$g of the peptide per gram of the animal weight. The placebo group was treated with proteins from *E.coli* contained in PBS 1X (*E. coli* proteins were obtained by the same procedure used for the purification of PACAP, with equivalent amount of contaminants present in the purified PACAP sample).

After applying the treatments by injection, the same amount of food was added to the three experimental groups, after 6 hours non ingested food was collected and new food was added. Turning to monitor the appetite for 22 hours the beginning of the experiment. The appetite was measured again, 22 hours of the beginning of the experiment.

The food collected from the bottom of each channel in each monitoring was oven-dried at 100 ° C for 20 hours and was weighed using an analytical balance. Food intake was calculated by determining the difference between the food added to the ponds (10 grams, with 20% humidity) and the food not eaten by the fish.

The tilapias injected with PACAP intraperitoneally, with the dose of 0,5 $\mu$g/gram of animal weight and the tilapias injected with the peptide GHRP-6, using the same method of administration, in a dose of 0,1 $\mu$g/gram of animal weight, demonstrated a significant increase in appetite ($p < 0.05$) compared with control group (Figure 8).

**Example 5. Evaluation of the effect of recombinant PACAP on the immune system.**

Juvenile *Claria gariepinus* were employed. Two groups were formed, with 10 animals in each group. The groups were acclimated in separated tanks with constant water recirculation, at a temperature of 28°C and with the photoperiod of 14 hours of light and 10 hours of darkness. The animals were fed 2 times per day, with equivalent rations to 5% of the total body weight in each tank. The peptide with the sequence SEQ ID No. 13, was applied by intraperitoneal injection 2 times per week in a dose of 0,1 $\mu$g gram animal weight.

Twenty days after the start of the experiment blood of the animals was extracted to measure the lysozyme and lectin levels in blood serum. The activity of lysozyme in the serum was determined by a turbidimetric method based on lysis of lyophilized *Micrococcus lysodeikticus.* Egg white lysozyme of chicken was used as a positive control. Samples of lysozyme were prepared in concentrations of 0, 1, 2, 4 and 8 mg / mL in phosphate buffer (0,05 M $NaHPO_4$ $12H_2O$, pH 6,2). The microorganism was prepared in a concentration of 3 mg / mL in phosphate buffer. 100 $\mu$l_positive control solutions were added in 96 well plates with a flat bottom and performed in duplo, and 100 $\mu$l_of serum was added in sixfold. In each of these wells 100 $\mu$l of suspension of the microorganism was added to a final volume of 200 $\mu$l in each well. The optical density (OD) was read immediately after adding the microorganism at A = 450 nm in a plate reader for 96 wells after 0, 2, 3, 5, 10, 15, 25, 35 and 45 min. Reactions were performed at approximately 22 ° C. The variation of 0.001 OD units per minute was considered as one Unit (U) of lysozyme. The units of lysozyme of the controls and the samples were calculated as follows: dividing the slope of the curve $\Delta DO_{450\ nm}$ vs t (min) (in its linear range) by 0,001. This results in a reference curve $U_{lisozima}$ vs mg /mL lysozyme from which the units of enzymatic activity of the samples are interpolated to derive their concentration. The results of the experiment showed that the fish treated with PACAP demonstrate a highly statistically significant ($p < 0,01$) increase in de level of lysozyme in serum compared to the placebo group (Table 3).

**Table 3: Lysozyme concentration ($\mu$g/mL) in the fish serum 20 days after the beginning of the experiment**

| LYSOZYME CONCENTRATION | PACAP TREATED GROUP | CONTROL GROUP |
|---|---|---|
| $\mu$g/mL | 21.765±5.438* | 7.828±8.393 |
| The concentration is shown as means ± S.D<br>* Student test. Indicates significant differences P<0.01 | | |

In order to determine the presence of lectin in blood serum, we set up a haemagglutination assay. Serial dilutions were performed in PBS 1X pH 7.2 divided in 100 $\mu$l serum in 96 well plates with a U-shaped bottom. Hereto, an equal volume of freshly prepared 2% erythrocyte suspension (rabbit in PBS) was added. Wells were incubated for 1 h at room temperature and the titer was read out visually. The activity of lectins was expressed as the reciprocal of the highest dilution showing complete hemoagglutination.

The fish treated with the peptide demonstrated an statistically significant increase in the level of lectins compared to the placebo group (p<0.05) (Table 4).

**Table 4: Titer of haemagglutinin activity (the reciprocal of the highest dilution showing complete agglutination) in the fish serum at 45 days from the beginning of the experiment.**

|  | PACAP TREATED GROUP | PLACEBO GROUP |
|---|---|---|
| TITER | 4* | 1 |
| Student test. * indicates a significant difference P<0.05 | | |

**Example 6. Experiment of growth stimulation in tilapia larvae by application of recombinant PACAP contained in the supernatant the culture of *P. pastoris.***

We made an experiment to evaluate the function of the recombinant *Clarias gariepinus* PACAP (SEQ ID No 14) contained in the supernatant of *P. pastoris* culture in the growth of the tilapia larvae. Three experimental groups of 50 larvae per group were formed. One group was treated with recombinant PACAP (SEQ ID No. 14) contained in *P. pastoris* culture supernatants. One group was treated with tilapia growth hormone (GH) and one group with a placebo. Larvae from each group were acclimated in separate channels 80L at a temperature of 280C and a photoperiod of 14 hours light and 10 hours of darkness and were fed with an amount of food according to the formula: Amount of food = # of animals X average weight (g) X 40% / 100. The treatments were performed by immersion in an hour and a half in a volume of 20 liters of water, three times a week for 30 days, with approximately 100 mg of peptide / liter of water.

Results showed that 5 weeks after the initiation of the experiment (35 days) the group treated with pACAP showed a significant increase in the average body weight compared to the placebo group (p <0.01). The group treated with growth hormone expressed in tilapia culture supernatant of P. pastoris showed also an increase in average body weight statistically significant placebo group (p <0.05) (Table 5).

**Table 5:Tilapia larvae weight in grams.**

| TIME (DAYS) | PACAP TREATED GROUP | GH TREATED GROUP | PLACEBO GROUP |
|---|---|---|---|
| 0 | 0.1013±0,0625 | 0.1140±0.0457 | 0.1040±0.0535 |
| 35 | 0. 9870±0.0525 | 0. 7875±0.0422 | 0.4566±0.0363 |
| Mass is shown as mean ± S.D | | | |

**Example 7. Experiment of growth stimulation and quality in larvae of the shrimp of the *Litopenaeus schmitti* species treated with recombinant PACAP contained in the supernatant of *Pichia pastoris.***

We used the shrimp's larvae of the *Litopenaeus schmitti* species. Two experimental groups were formed.,One group was treated with recombinant PACAP (SEQ ID No. 14) contained in the supernatant of *P. pastoris* culture and one group was treated with a placebo (non-transformed *P. pastoris* culture).

The larvae were cultivated in fiber glass tanks with a capacity of 100 L. The feeding was based on dyatomeas (*Chaetoceros gracilis*), the flagellated algae (*Tetraselmis suecica*) and Artemia nauplius (Aquatic Eco-Systems Inc.).

The abiotic growth factors were the following ones:

- illumination (24:00 UD)
- Constant aeration.
- Salinity of 34 ppm.
- Dissolved oxygen 5.2 ± 0.5 (in the larvae cycle).
- Recirculation after $PZ_{III}$ of the 80%

Four immersion baths were applied to the experimental groups, one every three days of 1 hour of duration.

We obtained as results that the group treated with the peptide showed a significant body weight increase compared with the control group (p<0.01) (Table 6).

**Table 6: Shrimp larvae weight in milligrams.**

| TIME (DAYS) | PACAP TREATED GROUP | CONTROL GROUP |
|---|---|---|
| 0 | 0.3045±0.0425 | 0.3273±0.0420 |
| 30 | 12.5034±0.0455 | 6.5325±0.0438 |
| Mass is shown as mean ± S.D | | |

We observed a higher homogeneity and better quality of the larvae (more branquial ramifications and rostral modifications) which is very important in the shrimp farming. The difference in the PL9 stage survival was greater than 40% in PACAP treated group.

**Example 8. Growth stimulation in juveniles of *Clarias gariepinus* using the formulation of recombinant PACAP in the fish diet.**

The recombinant PACAP (SEQ ID No. 14) contained in the supernatant of *Pichia pastoris* culture was concentrated and formulated in the nutritional fish diet in a concentration of approximately 5 mg/Kg of feed. The negative control was the diet formulated with the supernatant of culture of non transformed *P. pastoris.* The excperiment was carried out during 30 days. The recombinant PACAP (SEQ ID No. 14) included in the diet at the dose of 5 mg/Kg of feed resulted in an increase in body weight of the fish of approximately 30% compared with the placebo group, with highly significant statistical differences between the groups (p<0.01).

**Claims**

1. A method to increase the productivity of fish or crustaceans culture that comprises feeding or administering a Pituitary Adenylate Cyclase-Activating Polypeptide (PACAP) neuropeptide with the amino acid sequence identified as SEQ ID No 12, SEQ ID No 13 or SEQ ID No 14, to fish or crustaceans in culture in an amount effective to stimulate their growth or to increase their resistance against diseases or both.

2. A method according to claim 1, wherein the PACAP neuropeptide is used to increase the prolactin secretion and to improve the fish osmoregulation.

3. A method according to claim 1, wherein the PACAP neuropeptide is used to regulate the appetite in aquatic organisms.

4. A method according to claim 1, wherein the PACAP neuropeptide is used to improve the development of coloration in ornamental fish and crustaceans.

5. A method according to claim 1, wherein the PACAP neuropeptide is obtained by chemical synthesis.

6. A method according to claim 1, wherein the PACAP neuropeptide is obtained by recombinant technology.

7. A method according to claim 6, wherein the PACAP neuropeptide is used without purification, contained in the supernatant of ruptured *E. coli* expressing PACAP.

8. A method according to claim 6, wherein the PACAP neuropeptide is used without purification, contained in the culture supernatant of *P. pastoris* expressing PACAP.

9. A method according to claim 6, wherein the PACAP neuropeptide is used purified starting from recombinant production systems.

10. A method according to claim 1, wherein the PACAP neuropeptide is applied to fish or crustaceans by periodic injections every 3 days at the concentration of 0,1 $\mu$g/g of animal weight.

11. A method according to claim 1, wherein the PACAP neuropeptide is supplied to fish or crustaceans by immersion baths at intervals of 1 to 4 days in fresh water or sea water, in a concentration between 100 and 200 $\mu$g of the PACAP per liter of water.

12. A method according to claim 1, wherein the PACAP neuropeptide is supplied to fish or crustaceans as a formulated feed in a concentration of 5 mg of the PACAP per kg of feed.

**13.** A method according to claims 1 to 12, wherein the PACAP neuropeptide is supplied to tilapia *Orechromis sp.*

**14.** A method according to claims 1 to 12, wherein the PACAP neuropeptide is supplied to catfish *Claria sp..*

**15.** A method according to claims 1 to 12, wherein the PACAP neuropeptide is supplied to salmon *Salmon sp..*

**16.** A method according to claims 1 to 12, wherein the PACAP neuropeptide is supplied to shrimp *Penaus sp..*

**17.** A method according to claim 1, wherein the PACAP neuropeptide is supplied to fish or crustaceans to prevent or to treat infections caused by pathogenic agents.

**18.** The use of the PACAP neuropeptide with the amino acid sequence identified as SEQ ID No 12, SEQ ID No 13 or SEQ ID No 14 in the preparation of a composition to treat fish or crustaceans in culture to stimulate their growth.

**19.** The use of the PACAP neuropeptide with the amino acid sequence identified as SEQ ID No 12, SEQ ID No 13 or SEQ ID No 14 in the preparation of a composition to treat fish or crustaceans in culture to increase its resistance against diseases.

**20.** The use of the PACAP neuropeptide with the amino acid sequence identified as SEQ ID No 12, SEQ ID No 13 or SEQ ID No 14 in the preparation of a composition for the preventive or therapeutic treatment of an infection caused by pathogenic agents in fish or crustaceans in culture.

**21.** The use of the PACAP neuropeptide with the amino acid sequence identified as SEQ ID No 12, SEQ ID No 13 or SEQ ID No 14 to stimulate the fish and crustaceans growth in culture, to improve the productivity.

**22.** The use of the PACAP neuropeptide with the amino acid sequence identified as SEQ ID No 12, SEQ ID No 13 or SEQ ID No 14 to increase resistance of fish and crustaceans in culture to diseases, to improve the productivity.

**23.** The use of the PACAP neuropeptide with the amino acid sequence identified as SEQ ID No 12, SEQ ID No 13 or SEQ ID No 14 for the prevention or treatment of infections caused by pathogenic agents of fish and crustaceans in culture, to improve the productivity.

**Patentansprüche**

**1.** Verfahren zum Erhöhen der Produktivität einer Fisch- oder Krustazeen-Kultur, welches das Füttern oder Verabreichen eines Pituitary Adenylate Cyclase Activating Polypeptide (PACAP)-Neuropeptids mit der als SEQ ID Nr. 12, SEQ ID Nr. 13 oder SEQ ID Nr. 14 identifizierten Aminosäuresequenz an Fische oder Krustazeen in Kultur in einer zum Stimulieren ihres Wachstums oder zum Erhöhen ihrer Widerstandsfähigkeit gegen Krankheiten oder beides wirksamen Menge umfasst.

**2.** Verfahren gemäß Anspruch 1, wobei das Neuropeptid PACAP zum Erhöhen der Prolaktin-Sekretion und zum Verbessern der Osmoregulation der Fische verwendet wird.

**3.** Verfahren gemäß Anspruch 1, wobei das Neuropeptid PACAP zum Regulieren des Appetits bei Wasserorganismen verwendet wird.

**4.** Verfahren gemäß Anspruch 1, wobei das Neuropeptid PACAP zum Verbessern der Entwicklung einer Färbung bei Zierfischen und Krustazeen verwendet wird.

**5.** Verfahren gemäß Anspruch 1, wobei das Neuropeptid PACAP durch chemische Synthese erhalten wird.

**6.** Verfahren gemäß Anspruch 1, wobei das Neuropeptid PACAP durch rekombinante Technologie erhalten wird.

**7.** Verfahren gemäß Anspruch 6, wobei das Neuropeptid PACAP ohne Reinigung verwendet wird, das im Überstand aufgebrochener *E. coli,* die PACAP exprimieren, enthalten ist.

**8.** Verfahren gemäß Anspruch 6, wobei das Neuropeptid PACAP ohne Reinigung verwendet wird, das im Kulturüberstand von *P. pastoris,* die PACAP exprimieren, enthalten ist.

9. Verfahren gemäß Anspruch 6, wobei das Neuropeptid PACAP gereinigt verwendet wird, ausgehend von rekombinanten Produktionssystemen.

10. Verfahren gemäß Anspruch 1, wobei das Neuropeptid PACAP bei Fischen oder Krustazeen durch regelmäßige Injektionen alle 3 Tage bei der Konzentration von 0,1 $\mu$g/g Tiergewicht angewendet wird.

11. Verfahren gemäß Anspruch 1, wobei das Neuropeptid PACAP Fischen oder Krustazeen durch Tauchbäder in Abständen von 1 bis 4 Tagen in Süßwasser oder Meerwasser in einer Konzentration zwischen 100 und 200 $\mu$g PACAP pro Liter Wasser zugeführt wird.

12. Verfahren gemäß Anspruch 1, wobei das Neuropeptid PACAP Fischen oder Krustazeen als formuliertes Futter in einer Konzentration von 5 mg PACAP pro kg Futter zugeführt wird.

13. Verfahren gemäß den Ansprüchen 1 bis 12, wobei das Neuropeptid PACAP dem Tilapia *Orechromis sp.* zugeführt wird.

14. Verfahren gemäß den Ansprüchen 1 bis 12, wobei das Neuropeptid PACAP dem Wels *Claria sp.* zugeführt wird.

15. Verfahren gemäß den Ansprüchen 1 bis 12, wobei das Neuropeptid PACAP dem Lachs *Salmon sp.* zugeführt wird.

16. Verfahren gemäß den Ansprüchen 1 bis 12, wobei das Neuropeptid PACAP der Garnele *Penaus sp.* zugeführt wird.

17. Verfahren gemäß Anspruch 1, wobei das Neuropeptid PACAP Fischen oder Krustazeen zugeführt wird, um durch pathogene Agenzien verursachte Infektionen zu verhindern oder zu behandeln.

18. Verwendung des Neuropeptids PACAP mit der als SEQ ID Nr. 12, SEQ ID Nr. 13 oder SEQ ID Nr. 14 identifizierten Aminosäuresequenz bei der Herstellung einer Zusammensetzung zum Behandeln von Fischen oder Krustazeen in Kultur, um ihr Wachstum zu stimulieren.

19. Verwendung des Neuropeptids PACAP mit der als SEQ ID Nr. 12, SEQ ID Nr. 13 oder SEQ ID Nr. 14 identifizierten Aminosäuresequenz bei der Herstellung einer Zusammensetzung zum Behandeln von Fischen oder Krustazeen in Kultur, um ihre Widerstandsfähigkeit gegen Krankheiten zu erhöhen.

20. Verwendung des Neuropeptids PACAP mit der als SEQ ID Nr. 12, SEQ ID Nr. 13 oder SEQ ID Nr. 14 identifizierten Aminosäuresequenz bei der Herstellung einer Zusammensetzung zur präventiven oder therapeutischen Behandlung einer durch pathogene Agenzien bei Fischen oder Krustazeen in Kultur verursachten Infektion.

21. Verwendung des Neuropeptids PACAP mit der als SEQ ID Nr. 12, SEQ ID Nr. 13 oder SEQ ID Nr. 14 identifizierten Aminosäuresequenz zum Stimulieren des Wachstums von Fischen und Krustazeen in Kultur, um die Produktivität zu verbessern.

22. Verwendung des Neuropeptids PACAP mit der als SEQ ID Nr. 12, SEQ ID Nr. 13 oder SEQ ID Nr. 14 identifizierten Aminosäuresequenz zum Erhöhen der Widerstandsfähigkeit von Fischen und Krustazeen gegen Krankheiten, um die Produktivität zu verbessern.

23. Verwendung des Neuropeptids PACAP mit der als SEQ ID Nr. 12, SEQ ID Nr. 13 oder SEQ ID Nr. 14 identifizierten Aminosäuresequenz zur Vorbeugung oder Behandlung von Infektionen, die durch pathogene Agenzien von Fischen und Krustazeen in Kultur verursacht werden, um die Produktivität zu verbessern.

**Revendications**

1. Procédé pour augmenter la productivité d'une culture de poissons ou de crustacés, qui comprend l'alimentation ou l'administration d'un neuropeptide du type polypeptide d'activation de l'adénylcyclase pituitaire (PACAP), présentant la séquence d'acides aminés identifiée par la SEQ ID n° 12, la SEQ ID n° 13 ou la SEQ ID n° 14, à des poissons ou à des crustacés en culture en une quantité efficace pour stimuler leur croissance ou pour augmenter leur résistance aux maladies, voire les deux.

2. Procédé selon la revendication 1, dans lequel le neuropeptide PACAP est utilisé pour augmenter la sécrétion de prolactine et pour améliorer l'osmorégulation du poisson.

3. Procédé selon la revendication 1, dans lequel le neuropeptide PACAP est utilisé pour réguler l'appétit chez des organismes aquatiques.

4. Procédé selon la revendication 1, dans lequel le neuropeptide PACAP est utilisé pour améliorer le développement de la coloration des poissons et des crustacés d'ornement.

5. Procédé selon la revendication 1, dans lequel le neuropeptide PACAP est obtenu par synthèse chimique.

6. Procédé selon la revendication 1, dans lequel le neuropeptide PACAP est obtenu par une technologie recombinant.

7. Procédé selon la revendication 6, dans lequel le neuropeptide PACAP est utilisé sans purification, contenu dans le surnageant de cellules éclatées d'*E. coli* exprimant le PACAP.

8. Procédé selon la revendication 6, dans lequel le neuropeptide PACAP est utilisé sans purification, sous la forme contenue dans le surnageant de culture de *P. pastoris* exprimant le PACAP.

9. Procédé selon la revendication 6, dans lequel le neuropeptide PACAP est utilisé sous forme purifiée à partir de systèmes de production recombinants.

10. Procédé selon la revendication 1, dans lequel le neuropeptide PACAP est appliqué à des poissons ou à des crustacés par des injections périodiques tous les 3 jours à la concentration de 0,1 $\mu$g/g du poids de l'animal.

11. Procédé selon la revendication 1, dans lequel le neuropeptide PACAP est fourni à des poissons ou à des crustacés par le biais de bains d'immersion à des intervalles de 1 à 4 jours dans de l'eau douce ou dans de l'eau de mer, à une concentration allant de 100 à 200 $\mu$g de PACAP par litre d'eau.

12. Procédé selon la revendication 1, dans lequel le neuropeptide PACAP est fourni à des poissons ou à des crustacés sous la forme d'une alimentation formulée à une concentration de 5 mg de PACAP par kg de nourriture.

13. Procédé selon les revendications 1 to 12, dans lequel le neuropeptide PACAP est fourni aux tilapias de l'espèce *Oreochromis.*

14. Procédé selon les revendications 1 à 12, dans lequel le neuropeptide PACAP est fourni aux poissons-chats de l'espèce *Clarias.*

15. Procédé selon les revendications 1 à 12, dans lequel le neuropeptide PACAP est fourni aux saumons de l'espèce *Salmon.*

16. Procédé selon les revendications 1 à 12, dans lequel le neuropeptide PACAP est fourni aux crevettes de l'espèce *Penaeus.*

17. Procédé selon la revendication 1, dans lequel le neuropeptide PACAP est fourni à des poissons ou à des crustacés dans le but d'empêcher ou de traiter des infections provoquées par des agents pathogènes.

18. Utilisation du neuropeptide PACAP présentant la séquence d'acides aminés identifiée par la SEQ ID n° 12, la SEQ ID n° 13 ou la SEQ ID n° 14 dans la préparation d'une composition destinée à traiter des poissons ou des crustacés en culture dans le but de stimuler leur croissance.

19. Utilisation du neuropeptide PACAP présentant la séquence d'acides aminés identifiée par la SEQ ID n° 12, la SEQ ID n° 13 ou la SEQ ID n° 14 dans la préparation d'une composition destinée à traiter des poissons ou des crustacés en culture dans le but d'augmenter leur résistance vis-à-vis des maladies.

20. Utilisation du neuropeptide PACAP présentant la séquence d'acides aminés identifiée par la SEQ ID n° 12, la SEQ ID n° 13 ou la SEQ ID n° 14 dans la préparation d'une composition destinée à traiter de manière préventive ou thérapeutique une infection provoquée par des agents pathogènes chez des poissons ou des crustacés en culture.

21. Utilisation du neuropeptide PACAP présentant la séquence d'acides aminés identifiée par la SEQ ID n° 12, la SEQ ID n° 13 ou la SEQ ID n° 14, dans le but de stimuler la croissance de poissons et de crustacés en culture pour améliorer la productivité.

22. Utilisation du neuropeptide PACAP présentant la séquence d'acides aminés identifiée par la SEQ ID n° 12, la SEQ ID n° 13 ou la SEQ ID n° 14, dans le but d'augmenter la résistance de poissons et de crustacés en culture vis-à-vis de maladies, pour améliorer la productivité.

23. Utilisation du neuropeptide PACAP présentant la séquence d'acides aminés identifiée par la SEQ ID n° 12, la SEQ ID n° 13 ou la SEQ ID n° 14, dans le but de prévenir ou de traiter des infections provoquées par des agents pathogènes chez des poissons et des crustacés en culture, pour améliorer la productivité.

**Figure 1 A**

**Figure 1B**

Figure 2

Figure 3

**Figure 4A**

**Figure 4B**

**Figure 5**

**Figure 6**

**Figure 7**

**Figure 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5695954 A
- CU 2006000013 W

- CU 2005023120

### Non-patent literature cited in the description

- **Miyata et al.** Isolation of a novel 38 residue hypothalamic polypeptide which stimulates adenylate cyclase in pituitary cells. *Biochem. Biophys. Res. Commun.,* 1989, vol. 164, 567-574
- **Arimura ; Shioda.** Pituitary adenylate cyclase-activating polypeptide (PACAP) and its receptors: Neuroendocrine and endocrine interaction. *Front. Neuroendocrinol.,* 1995, vol. 16, 53-88
- **Hosoya et al.** Structure of the human pituitary adenylate cyclase-activating polypeptide (PACAP) gen. *Biochim. Biophys. Acta,* 1992, vol. 1129, 199-206
- **Montero et al.** Molecular evolution of the growth hormone-releasing hormone/pituitary adenylate cyclase-activating polypeptide gene family. Functional implication in the regulation of growth hormone secretion. *Journal of Molec. Endocrinol.,* 2000, vol. 25, 157-168
- **Sherwood et al.** The origin and function of the Pituitary Adenylate Cyclase-Activating Polypeptide (PACAP)/Glucagon Superfamily. *Endocrine Review,* 2000, vol. 21, 619-670
- **Chatterjee et al.** Genomic organization of the rat pituitary adenylate cyclase-activating polypeptide receptor gene. Alternative splicing within the 59-untranslated region. *J. Biol. Chem.,* 1997, vol. 272, 12122-12131
- **Hu et al.** Characterization and messenger ribonucleic acid distribution of a cloned pituitary adenylate cyclase-activating polypeptide type I receptor in the frog Xenopus laevis brain. *Endocrinol.,* 2000, vol. 141, 657-665
- **Anderson L. L. et al.** Growth Hormone Secretion: Molecular and Cellular Mechanisms and In Vivo Approaches. *Society for Experim. Biol. and Med.,* 2004, vol. 229, 291-302
- **Jarr et al.** Contrasting effects of pituitary adenylate cyclase activating polypeptide (PACAP) on in vivo and in vitro prolactin and growth hormone release in male rats. *Life Sci.,* 1992, vol. 51, 823-830

- **Radcliff et al.** Pituitary adenylate cyclase-activating polypeptide induces secretion of growth hormone in cattle. *Domestic. Animal. Endocrinol.,* 2001, vol. 21, 187-196
- **Sawangjaroen ; Curlewis.** Effects of pituitary adenylate cyclaseactivating polypeptide (PACAP) and vasoactive intestinal polypeptide (VIP) on prolactin, luteinizing hormone and growth hormone secretion in the ewe. *J. Neuroendocrinol.,* 1994, vol. 6, 549-555
- **Chlodera et al.** Effects of intravenously infused pituitary adenylate cyclase activating polypeptide on adenohypophyseal hormone secretion in normal men. *Clin. Neuroendocrinol.,* 1996, vol. 64, 242-246
- **Anderson et al.** Growth Hormone Secretion: Molecular and Cellular Mechanisms and In Vivo Approaches. *Society for Experim. Biol. and Med.,* 2004, vol. 229, 291-302
- **Ortmann et al.** Interactions of ovarian steroids with pituitary adenylate cyclase-activating polypeptide and GnRH in anterior pituitary cells. *Eur. J. Endocrinol.,* 1999, vol. 140, 207-214
- **Vaudry et al.** Pituitary adenylate cyclase-activating polypeptide and its receptors: from structure to functions. *Pharmacol. Rev.s,* 2000, vol. 52, 269-364
- **Sherwood et al.** The Origin and Function of the Pituitary Adenylate Cyclase-Activating olypeptide (PACAP). *Glucagon Superfamily Endocrine Reviews,* 2000, vol. 21 (6), 619-670
- **Leung et al.** Molecular cloning and tissue distribution of in pituitary adenylate cyclase-activating polypeptide (PACAP) the goldfish. *Rec. Progr. Mol. Comp. Endocrino/.,* 1999, 338-388
- **Fradinger ; Sherwood.** Characterization of the gene encoding both growth hormone-releasing hormone (GRF) and pituitary adenylate cyclase-activating polypeptide. *Mol. and Cell. Endocrinol.,* 2000, vol. 165, 211-219

- **Krueckl ; Sherwood.** Developmental expression, alternative splicing and gene copy number for the pituitary adenylate cyclase-activating polypeptide (PACAP) and growth hormone-releasing hormone (GRF) gene in raibow trout. *Molec. and Cell. Endocrinol.,* 2001, vol. 182, 99-108
- **Pullin et al.** Conference Proceeding. International Center for living Aquatic Resources Monagement, 1982, vol. 7, 432
- **Tschopp et al.** *Bio/Technology,* 1987, vol. 5, 1305-1308
- **Barr et al.** *Pharm. Eng.,* 1992, vol. 12, 48-51
- **Jensen.** Regulatory peptides and control of food intake in non-mammalian vertebrates. *Comp. Biochem. And Phisiol. Part A,* 2001, vol. 128, 471-479